# EUROPÄISCHE PATENTANMELDUNG

(11) **EP 4 387 122 A1**
(43) Veröffentlichungstag der Anmeldung: **19.06.2024**
(21) Anmeldenummer: 23217268.4
(22) Anmeldetag: 15.12.2023
(51) Int. Cl.: H04B 10/114, A61B 50/13, G08C 23/04, H04B 10/116

(54) **NETZWERKVORRICHTUNG ZUR DATENÜBERTRAGUNG IM OPERATIONSSAAL**

(30) Priorität: 15.12.2022 DE 102022133523
(71) Anmelder: Karl Storz SE & Co. KG, 78532 Baden-Württemberg Tuttlingen (DE)
(72) Erfinder: Bohring, Martin, 78532 Tuttlingen (DE)

(57) **Zusammenfassung**

Die Anmeldung betrifft eine Vorrichtung zur Datenkommunikation (10) für eine bewegliche Operationssaaleinrichtung (20), insbesondere einem Trolley, aufweisend lokale Netzwerkmittel (11) zur Verbindung von der Operationssaaleinrichtung (20) zugeordneten medizinischen Komponenten (12), und eine mit den lokalen Netzwerkmitteln (11) verbundene Netzwerkschnittstelle (13) zur vorzugsweise bidirektionalen Datenkommunikation mit einem externes Netzwerk und/oder einer externen Datenbank (30), wobei die Netzwerkschnittstelle (13) zur Datenkommunikation eine Light-Fidelity-Schnittstelle ist.

Weiterhin betrifft die Erfindung eine bewegliche Operationssaaleeinrichtung, ein System und ein Verfahren zur Datenkommunikation zwischen einer beweglichen Operationssaaleinrichtung und einem stationären lokalen Krankenhausnetzwerk.

## Beschreibung

Die Erfindung betrifft eine Netzwerkvorrichtung zur Datenübertragung für eine bewegliche Operationssaaleinrichtung, insbesondere einem mobilen Trolley.

Aus dem Stand der Technik sind sogenannte Trolleys bekannt. Hierbei handelt es sich um einen mobilen medizinischen Wagen, der medizinische Geräte oder Komponenten transportiert, aufbewahrt und für die Benutzung zur Verfügung stellt. Die Trolleys als Träger der medizinischen Geräte oder Komponenten können dabei je nach vorzunehmender Behandlung oder Operation zwischen den verschiedenen Operationssälen aber auch innerhalb eines Operationssaals verschoben und positioniert werden. Dies geschieht mit dem Ziel, die Anzahl der nötigen Trolleys zu minieren, aber auch um eine optimale ergonomische Position der Trolleys während der Behandlung oder Operation bereitzustellen.

Ursprünglich war an den jeweils gewünschten Standorten des oder der Trolleys lediglich eine Spannungsversorgung vorgesehen. In modernen Operationssälen ist aber eine umfassendere Datenkommunikation aufweisend eine Vielzahl an zusätzlichen Daten, wie beispielsweise Einstellwerte für die medizinischen Geräte, Zustandsinformationen der Geräte, Live-Video-Daten und/oder eine elektronische Dokumentation einer Behandlung von und zum Trolley wünschenswert. Hieraus ergibt sich eine notwendige relativ hohe Bandbreite für die Übertragung der gewünschten Daten von und zum Trolley, insbesondere für die Anbindung an ein externes Netzwerk und/oder eine externe Datenbank wie beispielsweise ein lokales Kliniknetzwerk, so dass gewisse Datenübertragungsmöglichkeiten wie beispielsweise eine Infrarotschnittstelle am Trolley keine ausreichende Bandbreite aufweisen.

Aus dem Stand der Technik sind hierzu Netzwerkschnittstellen für eine bewegliche Operationssaaleinrichtung wie einem Trolley bekannt, welche zur kabelgebundenen Anbindung an ein internes und/oder externes Netzwerk oder Datenbanken per lokalem Netzwerk bzw. Local Area Network (LAN) oder zur kabellosen Anbindung per Wireless Local Area Network (WLAN) ausgebildet sind.

Eine kabelgebundene Anbindung des Trolleys an ein internes und/oder externes Netzwerk hat hierbei den Vorteil einer sicheren Datenübertragung, führt allerdings bei der Installation der beweglichen Operationssaaleinrichtung zu Einschränkungen. Insbesondere besteht im medizinischen Kontext gerade bei Anwendungen im Operationssaal der Wunsch einer möglichst freien Verfahrbarkeit des Trolleys im Raum, um eine gewünschte Positionierung relativ zu einem Patienten einnehmen und bei Bedarf flexibel anpassen zu können. Die dabei notwendige Kabelanbindung schränkt diese freie Positionierung ein und kann zudem unerwünschte Stolperfallen im Operationssaal darstellen. Die an sich bekannte drahtlose Anbindung eines Trolleys per WLAN-Schnittstelle ermöglicht zwar eine freie Positionierung im Raum, stellt allerdings eine unsichere und oftmals instabile Datenanbindung bereit. Insbesondere kann es bei der Regelung des geteilten Zugriffs in einem Wireless Local Area Network zu Verzögerungen kommen, was zu einer unerwünschten erhöhten nicht deterministischen Latenz führt. Dies kann gerade bei der Übertragung von Live-Video-Signalen besonders kritisch sein, da für endoskopische Operationen eine genaue Hand Augen Koordination des behandelnden Chirurgen notwendig ist. Zudem kann es aufgrund der Ausbreitung der weitreichenden WLAN-Signale auch durch Wände hindurch ohne entsprechende Abschirmmaßnahmen zu ungewünschten Gerätebedienungen kommen. Entsprechende Nachteile gelten für eine Datenanbindung des Trolleys bzw. einer beweglichen Operationssaaleinrichtung über die Datenkommunikationsstandards Bluetooth oder Zigbee.

Die Erfindung stellt sich nunmehr die Aufgabe basierend auf dem bekannten Stand der Technik eine verbesserte Vorrichtung zur Datenübertragung für eine bewegliche Operationssaaleinrichtung wie einem Trolley bereitzustellen, welche einerseits eine möglichst freie Positionierbarkeit der beweglichen Operationssaaleinrichtung ermöglicht, und gleichzeitig eine sichere Übertragung von Daten unter Gewährleistung einer ausreichend hohen Bandbreite bereitstellt. Gelöst wird diese Aufgabe durch die Gegenstände der unabhängigen Ansprüche. Die abhängigen Ansprüche beschreiben vorteilhafte Weiterbildungen der vorliegenden Erfindung.

Gemäß einem ersten Aspekt betrifft die Erfindung eine Vorrichtung zur Datenkommunikation für eine bewegliche Operationssaaleinrichtung, insbesondere einem Trolley, aufweisend lokale Netzwerkmittel zur Verbindung von der Operationssaaleinrichtung zugeordneten medizinischen Komponenten, und eine mit den lokalen Netzwerkmitteln verbundene Netzwerkschnittstelle zur vorzugsweise bidirektionalen Datenkommunikation mit einem externes Netzwerk und/oder einer externen Datenbank, dadurch gekennzeichnet, dass die Netzwerkschnittstelle zur Datenkommunikation eine Light-Fidelity-Schnittstelle ist.

Light-Fidelity ist dabei eine an sich bekannte drahtlose Kommunikationstechnologie, die Licht zur Übertragung von Daten und Positionen zwischen Geräten nutzt, und welche in der Lage ist, Daten mit hoher Geschwindigkeit über das sichtbare Licht, das ultraviolette und/oder das infrarote Spektrum zu übertragen. Für die optische Datenübertragung werden üblicherweise LED-Lampen verwendet.

Durch die Ausbildung der Netzwerkschnittstelle der erfindungsgemäß0en Vorrichtung als Light-Fidelity-Schnittstelle wird eine sehr sichere Datenübertragung ermöglicht. Insbesondere wird eine abhörsichere und von elektromagnetischen Störfeldern unbeeinflussbare Möglichkeit der Datenübertragung bereitgestellt, welche zudem für die gewünschte Datenübertragung im Operationsbereich eine ausreichende Bandbreite bereitstellt. Weiterhin ermöglicht die erfindungsgemäße Vorrichtung eine Bereitstellung der Kommunikationsschnittstelle ohne aufwändige bauliche Maßnahmen im Operationssaal.

In einer vorteilhaften Ausführungsform umfassen die lokalen Netzwerkmittel ein lokales Netzwerk (LAN) zur Verbindung einzelner, der Operationssaaleinrichtung zugeordneter medizinischer Komponenten. So können die Netzwerkmittel beispielsweise eine Endoskopiekamera und/oder weitere medizinische Komponenten, welche auf der beweglichen Operationssaaleinrichtung angeordnet sind, miteinander und/oder mit der Light-Fidelity-Schnittstelle verbinden. Die Light-Fidelity-Schnittstelle ist vorteilhaft ein an sich bekanntes Light-Fidelity Modem. Dieses umfasst insbesondere Mittel zur Emission und Detektion von optischen Signalen zur bidirektionalen Datenkommunikation, insbesondere entlang einer optischen Achse x.

Die Light-Fidelity-Schnittstelle bzw. das Light-Fidelity-Modem ist vorteilhaft zum selektiven Zusammenwirken mit wenigstens einem, vorzugsweise einer Mehrzahl von Light-Fidelity-Hotspot(s) zur bidirektionalen Datenkommunikation mit einem externen Krankenhaus-Netzwerk, insbesondere einem lokalen Netzwerk (LAN), ausgebildet. Die Light-Fidelity-Hotspots umfassen vorzugsweise ebenfalls Mittel zur Emission und Detektion von optischen Signalen zur bidirektionalen Datenkommunikation, insbesondere entlang einer optischen Achse.

Die Light-Fidelity-Schnittstelle ist dabei vorteilhaft derart in oder an der Vorrichtung angeordnet, dass eine Emission und/oder ein Empfang von optischen Signalen zur Datenkommunikation im Wesentlichen in Vertikalrichtung erfolgt. Dies bedeutet, dass die optische Achse der Light-Fidelity-Schnittstelle, entlang welcher die optische Datenübertragung bzw. -kommunikation erfolgt, im Wesentlichen vertikal ausgerichtet bzw. angeordnet ist.

In einem weiteren Aspekt betrifft die Erfindung eine Operationssaaleinrichtung aufweisend Verstell- und/oder Verfahrmittel zur vorzugsweisen freien Bewegung der Operationsaaleinrichtung und eine erfindungsgemäße Vorrichtung zur Datenkommunikation wie vorgehend beschrieben, wobei die Datenkommunikationsvorrichtung derart an der Operationssaaleinrichtung angeordnet ist, dass diese zur optischen Datenkommunikation in Vertikalrichtung ausgerichtet ist.

Die bewegliche Operationssaaleinrichtung ist vorteilhaft ein verfahrbarer Trolley, insbesondere ein Endoskopie-, Mikroskopie-, HF- und/oder Roboter-Trolley. Der Trolley weist hierbei wenigstens eine, bevorzugt eine Mehrzahl von darauf angeordneten medizinischen Komponenten auf, wie beispielsweise ein Endoskop und/oder ein Mikroskop. Die bewegliche Operationssaaleinrichtung kann ebenfalls ein beweglicher Operationstisch sein oder diesen umfassen.

Die Light-Fidelity-Schnittstelle ist vorteilhaft an einer oberen oder unteren Oberfläche der beweglichen Operationsaaleinrichtung angeordnet. Hierdurch wird eine vertikale optische Datenkommunikation mit einem jeweiligen Light-Fidelity-Hotspot ermöglicht, welcher vorzugsweise in Vertikalrichtung oberhalb oder unterhalb der beweglichen Operationsaaleinrichtung angeordnet ist. Die Light-Fidelity-Schnittstelle kann dabei in einer Aufnahme bzw. Öffnung, welche in der oberen oder unteren Oberfläche der Operationsaaleinrichtung ausgebildet ist, aufgenommen und somit von der jeweiligen Oberfläche zurückversetzt angeordnet sein

In einem weiteren Aspekt betrifft die Erfindung ein System zur Datenkommunikation für einen Operationssaal, aufweisend wenigstens eine bewegliche Operationseinrichtung wie vorgehend beschrieben, sowie wenigstens einen, bevorzugt mehrere damit selektiv zur Datenkommunikation zusammenwirkende und vorzugsweise fest im Operationssaal positionierte oder positionierbare Light-Fidelity-Hotspots.

Die Light-Fidelity-Hotspots sind dabei mit einem Krankenhausnetzwerk, vorzugsweise einem lokalen Netzwerk (LAN), verbunden. Dieses kann wenigstens einen Datenspeicher und/oder eine Servereinheit umfassen. Das Krankenhausnetzwerk kann weiterhin mit einer Benutzerschnittstelle, umfassend beispielsweise ein Display und/oder Eingabemittel wie eine Tastatur und/oder eine Maus gekoppelt sein oder diese umfassen.

Vorteilhaft sind die Light-Fidelity-Hotspots in oder an einem vordefinierten Decken- und/oder Bodenbereich des Operationssaals angeordnet und zur optischen vorzugsweise bidirektionalen Datenkommunikation mit der Operationssaaleinrichtung in Vertikalrichtung ausgebildet.

Die Light-Fidelity-Hotspots sind vorteilhaft in unterschiedlichen räumlichen Bereichen oder Zonen des Operationssaals angeordnet oder anordenbar, zur unterschiedlichen Positionierung der beweglichen Operationseinrichtung im Operationssaal. Weiterhin können auch mehrere Light-Fidelity-Hotspots in einem vordefinierten Bereich oder einer vordefinierten Zone des Operationssaals angeordnet sein.

In einer vorteilhaften Weiterbildung umfasst das System eine Steuerungseinrichtung, welche ausgebildet ist, eine Positionserfassung der beweglichen Operationssaaleinrichtung im Operationssaal mittels Erkennung einer selektiven Zusammenwirkung der Light-Fidelity-Schnittstelle der Operationssaaleinrichtung mit einem jeweiligen Light-Fidelity-Hotspot in einem vordefinierten räumlichen Bereich oder einer vordefinierten Zone des Operationssaals durchzuführen. Insbesondere kann die Steuerungseinrichtung hierbei eine Tracking- bzw. Erfassungseinrichtung zur Positionserfassung der beweglichen Operationssaaleinrichtung im Operationssaal ausbilden. Weiterhin kann die Steuerungseinrichtung ausgebildet sein, eine jeweilige Datenkommunikation, insbesondere zwischen der beweglichen Operationssaaleinrichtung und einem Krankenhausnetzwerk, abhängig von der jeweils erkannten Position der beweglichen Operationssaaleinrichtung im Operationssaal bzw. von dieser zugeordneten Positionsdaten durchzuführen und/oder anzupassen.

In einem weiteren Aspekt betrifft die Erfindung ein Verfahren zur Datenkommunikation zwischen einer beweglichen Operationssaaleinrichtung, insbesondere einem Trolley, und einem stationären lokalen Krankenhausnetzwerk, umfassend die Schritte:
- Bereitstellen einer Light-Fidelity-Schnittstelle in einer beweglichen Operationssaaleinrichtung,
- Bereitstellen wenigstens eines, bevorzugt einer Mehrzahl von Light-Fidelity-Hotspot(s) in einem Operationssaal, welche mit dem Krankenhausnetzwerk verbunden sind,
- Positionieren der beweglichen Operationssaaleinrichtung in einem optischen Erfassungsbereich eines Light-Fidelity-Hotspot(s) derart, dass die Light-Fidelity-Schnittstelle der Operationssaaleinrichtung zur Datenkommunikation entlang einer optischen und vorzugsweise vertikalen Achse des Light-Fidelity-Hotspots ausgerichtet ist.

Vorteilhaft weist das Verfahren den weiteren Schritt der Bereitstellung einer Steuerungseinrichtung auf, welche eine Positionierung der beweglichen Operationssaaleinrichtung in einem räumlichen Bereich eines jeweiligen Light-Fidelity-Hotspots durch Erkennung und/oder Auswertung einer Datenkommunikation zwischen der Light-Fidelity-Schnittstelle der Operationssaaleinrichtung und dem jeweiligen Light-Fidelity-Hotspot erfasst und vorzugsweise in einer Datenbank des Krankenhausnetzwerks wenigstens temporär abspeichert. Hierbei erfolgt eine Erkennung einer Positionierung der beweglichen Operationssaaleinrichtung in einem vordefinierten räumlichen Bereich des Operationssaals mittels Detektion eines visuellen Datensignals zwischen der Light-Fidelity-Schnittstelle und dem Light-Fidelity-Hotspot. Ein jeweiliger Light-Fidelity-Hotspot ist vorteilhaft in oder an einem oder mehreren Deckenabschnitten des Operationssaals, in einer Beleuchtungseinheit des Operationssaals und/oder in einer Operationsleuchte angeordnet.

Durch die Positionserfassung der Operationssaaleinrichtung wird unter anderem eine effektive Vorbereitung einer Operation oder Behandlung ermöglicht. Dies insbesondere, da dadurch bedingt, dass der Trolley sich potentiell in unterschiedlichen räumlichen Bereichen oder sogar in unterschiedlichen Operationssälen befinden könnte, die Kenntnis des genauen Standorts und die Positionierung am gewünschten Standort die Vorbereitung einer Behandlung für die Benutzer vereinfacht.

Die Datenkommunikation zwischen der Light-Fidelity-Schnittstelle der beweglichen Operationssaaleinrichtung und einem jeweiligen Light-Fidelity-Hotspot in einem Operationssaal umfasst vorteilhaft Einstellwerte, Zustandsinformationen und/oder Live-Video-Daten von wenigstens einer, der Operationssaaleinrichtung zugeordneter, medizinischer Komponente, beispielsweise einem Endoskop oder einem Operationsroboter.

Weiter vorteilhaft umfasst das Verfahren den Schritt der Anpassung der Datenkommunikation, insbesondere der übertragenen Daten oder eines Teils der übertragenen Daten basierend auf der jeweiligen, erkannten Positionierung der Operationssaaleinrichtung.

Zur Vermeidung von Wiederholungen wird auf die obige Beschreibung der erfindungsgemäßen Vorrichtung und des erfindungsgemäßen Systems verwiesen. Die diesbezüglich offenbarten Merkmale sollen auch als für das erfindungsgemäße Verfahren offenbart gelten und beanspruchbar sein und umgekehrt.

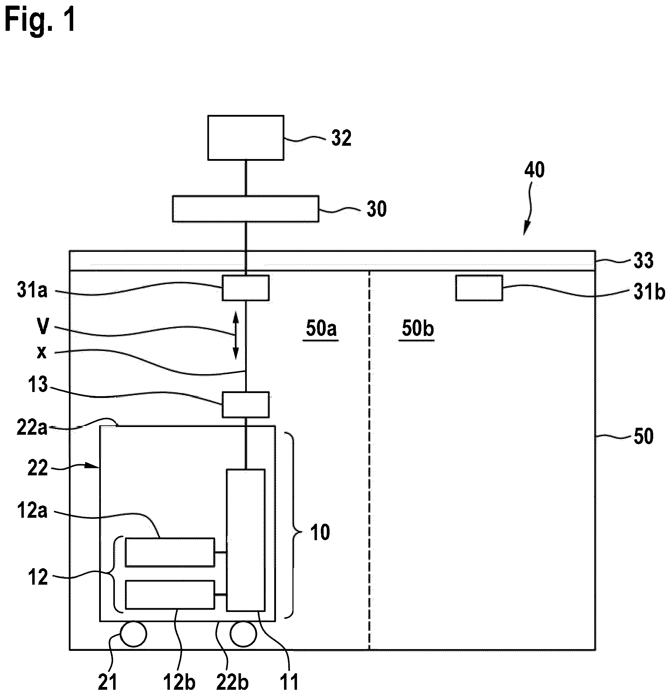

Einzelheiten, vorteilhafte Wirkungen und Details der vorliegenden Erfindung werden nachfolgend anhand der rein schematischen, lediglich beispielhaften Figur 1 erläutert. Diese zeigt ein erfindungsgemäßen System 40 zur Datenkommunikation für bzw. in einem Operationssaal 50, aufweisend eine beweglichen Operationssaaleinrichtung 20, vorliegend ein Trolley, und eine darin angeordnete erfindungsgemäße Vorrichtung zur Datenkommunikation 10. Der Trolley umfasst dabei Verfahrmittel 21, insbesondere Rollen, zur freien Positionierung des Trolleys im Operationssaal 50.

Die erfindungsgemäße Vorrichtung 10 umfasst lokale Netzwerkmittel 11, insbesondere ein lokales Netzwerk (LAN), welches zur Verbindung von medizinischen Komponenten 12 dient, die dem Trolley 20 zugeordnet bzw. in diesem angeordnet sind. Die medizinischen Komponenten 12 sind beispielsweise eine Endoskopiekamera 12a, eine mit dieser verbundene Eingabe- und/oder Displayeinheit 12b oder weitere Geräte. Die medizinischen Komponenten 12 können auch einen Operationsroboter wie beispielsweise einen Da-Vinci-Roboter umfassen.

Die Vorrichtung 10 umfasst weiterhin eine mit den Netzwerkmitteln 11 verbundene Netzwerkschnittstelle 13, welche zur bidirektionalen Datenkommunikation mit einem externen Netzwerk und/oder einer externen Datenbank, insbesondere eines lokalen Krankenhausnetzwerks 30 ausgebildet ist. Die Netzwerkschnittstelle 13 ist dabei erfindungsgemäß eine Light-Fidelity-Schnittstelle, welche an, auf oder in einer oberen Oberfläche 22a oder unteren Oberfläche 22b des Trolleys 20 angeordnet sein kann. Die Netzwerkschnittstelle 13 ist dabei derart an der Operationssaaleinrichtung 20 angeordnet ist, dass diese zur optischen Datenkommunikation in Vertikalrichtung V ausgerichtet ist.

Das System 40 umfasst weiterhin mehrere vorzugsweise fest im Operationssaal 50 positionierte Light-Fidelity-Hotspots 31a,b. Diese sind in vordefinierten räumlichen Bereichen oder Zonen 50a,50b des Operationssaals 50, insbesondere in oder an einem jeweiligen Deckenbereich 30, angeordnet und zur Datenkommunikation mit der Operationssaaleinrichtung 20 in Vertikalrichtung V ausgebildet.

Sobald der Trolley 20 mit der daran angeordneten Light-Fidelity-Schnittstelle 13 in einen optischen Erfassungsbereich entlang einer optischen Achse x eines jeweiligen Light-Fidelity-Hotspots 31a,b positioniert wird, kann in Vertikalrichtung V eine Datenkommunikation zwischen der Light-Fidelity-Schnittstelle 13 und dem jeweiligen Hotspot 31a,b zur bidirektionalen Datenübertragung mittels Light-Fidelity-Standard (Li-Fi) erfolgen. Durch die hergestellte Li-Fi Verbindung kann eine sichere und kabellose Datenübertragung wie beispielsweise von Einstellwerten, Zustandsinformationen und/oder Live-Video-Daten von wenigstens einer, der Operationssaaleinrichtung 20 zugeordneter, medizinischer Komponenten 12 und dem Krankenhausnetzwerk 30 bzw. den damit verbundenen Komponenten erfolgen.

Das System umfasst vorzugsweise weiterhin eine Steuerungseinrichtung 32, welche ausgebildet ist, eine Positionserfassung der beweglichen Operationssaaleinrichtung 20 im Operationssaal 50 mittels Erkennung einer Zusammenwirkung der Light-Fidelity-Schnittstelle 13 der Operationssaaleinrichtung 20 mit einem jeweiligen Light-Fidelity-Hotspot 31a,b durchzuführen. Hierdurch kann ein Tracking des Trolleys 20 im Operationssaal 50 erfolgen, bei welchem durch eine jeweils hergestellte Li-Fi Verbindung zwischen dem Trolley 20 und einem jeweiligen Hotspot 31a,b auf die jeweilige Position des Trolleys 20 durch Kenntnis der räumlichen Anordnung des jeweiligen Hotspots 31a,b geschlossen werden kann. Die jeweiligen Hotspots 31a,b sind dabei vorzugsweise in unterschiedlichen räumlichen Bereichen oder Zonen 50a,b des Operationssaal angeordnet. Weiter vorteilhaft kann die Steuerungseinrichtung 32 zur Anpassung der Datenkommunikation, insbesondere der übertragenen Daten oder eines Teils der übertragenen Daten basierend auf der jeweiligen, erkannten Positionierung der Operationssaaleinrichtung 20 ausgebildet sein.

### Bezugszeichenliste

- 10: Vorrichtung zur Datenkommunikation
- 11: lokale Netzwerkmittel
- 12: medizinische Komponenten
- 13: Netzwerkschnittstelle LIFI
- 20: beweglichen Operationssaaleinrichtung
- 21: Verstell- und/oder Verfahrmittel
- 22a,b: obere, untere Oberfläche
- 30: Krankenhausnetzwerk
- 31a,b: Light-Fidelity-Hotspot
- 32: Steuerungseinrichtung
- 33: Deckenbereich
- 40: System
- 50: Operationssaal
- 50a,b: räumliche Bereiche, Zonen

- V: Vertikalrichtung
- x: optische Achse

## Patentansprüche

1. Vorrichtung zur Datenkommunikation (10) für eine bewegliche Operationssaaleinrichtung (20), insbesondere einem Trolley, aufweisend lokale Netzwerkmittel (11) zur Verbindung von der Operationssaaleinrichtung (20) zugeordneten medizinischen Komponenten (12), und eine mit den lokalen Netzwerkmitteln (11) verbundene Netzwerkschnittstelle (13) zur vorzugsweise bidirektionalen Datenkommunikation mit einem externes Netzwerk und/oder einer externen Datenbank (30), **dadurch gekennzeichnet, dass** die Netzwerkschnittstelle (13) zur Datenkommunikation eine Light-Fidelity-Schnittstelle (Li-Fi) ist.

2. Vorrichtung nach Anspruch 1, **dadurch gekennzeichnet, dass** die lokalen Netzwerkmittel (11) ein lokales Netzwerk (LAN) zur Verbindung einzelner, der Operationssaaleinrichtung (20) zugeordneter medizinischer Komponenten (12) umfassen, und/oder dass die lichtbasierte Light-Fidelity-Schnittstelle (13) ein Light-Fidelity Modem ist.

3. Vorrichtung nach Anspruch 1 oder 2, **dadurch gekennzeichnet, dass** die lichtbasierte Light-Fidelity-Schnittstelle (13) zum selektiven Zusammenwirken mit wenigstens einem, vorzugsweise einer Mehrzahl von Light-Fidelity-Hotspot(s) (31) zur bidirektionalen Datenkommunikation mit einem externen Krankenhaus-Netzwerk (30), insbesondere einem lokalen Netzwerk (LAN), ausgebildet ist.

4. Vorrichtung nach einem der vorhergehenden Ansprüche, **dadurch gekennzeichnet, dass** die Light-Fidelity-Schnittstelle (13) derart in oder an der Vorrichtung (10) angeordnet ist, dass eine Emission und/oder der Empfang von optischen Signalen zur Datenkommunikation im Wesentlichen in Vertikalrichtung (V) erfolgt.

5. Operationssaaleinrichtung (20) aufweisend Verstell- und/oder Verfahrmittel (21) zur vorzugsweisen freien Bewegung der Operationsaaleinrichtung und eine Vorrichtung zur Datenkommunikation (10) gemäß einem der vorhergehenden Ansprüche, wobei die Datenkommunikationsvorrichtung (10) derart an der Operationssaaleinrichtung (20) angeordnet ist, dass diese zur optischen Datenkommunikation in Vertikalrichtung (V) ausgerichtet ist.

6. Operationssaaleinrichtung (20) nach Anspruch 5, **dadurch gekennzeichnet, dass** die Light-Fidelity-Netzwerkschnittstelle (13) an einer oberen oder unteren Oberfläche (22a,22b) der Operationsaaleinrichtung (20) angeordnet ist.

7. Operationssaaleinrichtung nach Anspruch 5 oder 6, **dadurch gekennzeichnet, dass** die Operationssaaleinrichtung (20) ein Endoskopie-, Mikroskopie-, HF- und/oder Roboter-Trolley, oder ein Operationstisch ist.

8. System (40) zur Datenkommunikation für einen Operationssaal (50), aufweisend wenigstens eine bewegliche Operationseinrichtung (20) nach einem der Ansprüche 5 bis 7, sowie wenigstens einen, bevorzugt mehrere damit selektiv zur Datenkommunikation zusammenwirkende und vorzugsweise fest im Operationssaal (50) positionierte oder positionierbare Light-Fidelity-Hotspot(s) (31).

9. System nach Anspruch 8, **dadurch gekennzeichnet, dass** der oder die Light-Fidelity-Hotspot(s) (31) in einem vordefinierten Decken-und/oder Bodenbereich des Operationssaals (50) angeordnet sind und zur Datenkommunikation mit der Operationssaaleinrichtung (20) in Vertikalrichtung (V) ausgebildet sind.

10. System nach Anspruch 8 oder 9, **dadurch gekennzeichnet, dass** die Light-Fidelity-Hotspots (31) in unterschiedlichen räumlichen Bereichen oder Zonen (50a,50b) des Operationssaals (50) angeordnet oder anordenbar sind.

11. System nach einem der Ansprüche 8 bis 10, **dadurch gekennzeichnet, dass** das System (40) eine Steuerungseinrichtung (32) umfasst, welche ausgebildet ist, eine Positionserfassung der beweglichen Operationssaaleinrichtung (20) im Operationssaal (50) mittels Erkennung einer selektiven Zusammenwirkung der Light-Fidelity-Schnittstelle (13) der Operationssaaleinrichtung (20) mit einem jeweiligen Light-Fidelity-Hotspot (31) durchzuführen.

12. Verfahren zur Datenkommunikation zwischen einer beweglichen Operationssaaleinrichtung (20), insbesondere einem Trolley, und einem stationären lokalen Krankenhausnetzwerk (30), umfassend die Schritte:
- Bereitstellen einer Light-Fidelity-Schnittstelle (13) in einer beweglichen Operationssaaleinrichtung (20),
- Bereitstellen wenigstens eines, bevorzugt einer Mehrzahl von Light-Fidelity-Hotspot(s) (31) in einem Operationssaal (50), welche mit dem Krankenhausnetzwerk (30) verbunden sind,
- Positionieren der beweglichen Operationssaaleinrichtung (20) in einem optischen Erfassungsbereich eines Light-Fidelity-Hotspot(s) (31) derart, dass die Light-Fidelity-Schnittstelle (13) der Operationssaaleinrichtung (20) zur Datenkommunikation entlang einer optischen und vorzugsweise vertikalen Achse (x) des Light-Fidelity-Hotspots (31) ausgerichtet ist.

13. Verfahren nach Anspruch 12, **dadurch gekennzeichnet, dass** das Verfahren den weiteren Schritt der Bereitstellung einer Steuerungseinrichtung (32) umfasst, welche eine Positionierung der beweglichen Operationssaaleinrichtung (20) in einem räumlichen Bereich eines jeweiligen Light-Fidelity-Hotspots (31) durch Erkennung und/oder Auswertung einer Datenkommunikation zwischen der Light-Fidelity-Schnittstelle (13) der Operationssaaleinrichtung (20) und dem jeweiligen Light-Fidelity-Hotspot (31) erfasst und vorzugsweise in einer internen Datenbank wenigstens temporär abspeichert.

14. Verfahren nach Anspruch 13, **dadurch gekennzeichnet, dass** das Verfahren den weiteren Schritt der Anpassung der Datenkommunikation, insbesondere der übertragenen Daten oder eines Teils der übertragenen Daten basierend auf der jeweiligen, erkannten Positionierung der Operationssaaleinrichtung (20) umfasst.

15. Verfahren nach einem der Ansprüche 12 bis 14, **dadurch gekennzeichnet, dass** die vorzugsweise bidirektionale Datenkommunikation zwischen der Light-Fidelity-Schnittstelle (13) der beweglichen Operationssaaleinrichtung (20) und einem jeweiligen Light-Fidelity-Hotspot (31) in einem Operationssaal (50) Einstellwerte, Zustandsinformationen und/oder Live-Video-Daten von wenigstens einer, der Operationssaaleinrichtung (20) zugeordneter, medizinischer Komponente (12), beispielsweise einem Endoskop oder einem Operationsroboter, umfasst.

16. Verfahren nach einem der Ansprüche 12 bis 15, **dadurch gekennzeichnet, dass** ein jeweiliger Light-Fidelity-Hotspot (31) in oder an einem oder mehreren Deckenabschnitten (30) des Operationssaals (50), in einer Beleuchtungseinheit des Operationssaals (50) und/oder in einer Operationsleuchte angeordnet ist.
